# EUROPÄISCHE PATENTSCHRIFT

(11) **EP 1 043 037 B1**
(45) Veröffentlichungstag und Bekanntmachung des Hinweises auf die Patenterteilung: **29.11.2006**
(21) Anmeldenummer: 00104742.2
(22) Anmeldetag: 04.03.2000
(51) Int. Cl.: A61M 5/00, A61M 5/315, A61M 5/178

(54) **Injektionsvorrichtung mit einem Pen**
A pen-type medicament injector
Injecteur de medicament du type stylo

(30) Priorität: 17.03.1999 DE 29904864 U
(43) Veröffentlichungstag der Anmeldung: 11.10.2000
(73) Patentinhaber: B. BRAUN MELSUNGEN AG, 34212 Melsungen (DE)
(72) Erfinder: Hartmann, Michael, 34212 Melsungen (DE)
(74) Vertreter: Selting, Günther

(56) Entgegenhaltungen:
- EP-A- 0 879 610
- WO-A-97/33638
- WO-A-99/02210
- US-A- 4 978 335
- US-A- 5 720 733
- US-A- 5 777 715

## Beschreibung

Die Erfindung betrifft eine Injektionsvorrichtung mit einem stiftförmigen Gehäuse, das eine handbetätigte Dosiermengen-Einstellvorrichtung aufweist.

Zum Spritzen von Insulin durch die Patienten sind Injektionsvorrichtungen mit stiftförmigem Gehäuse bekannt, bei denen die Injektionsmenge mit einer handbetätigten Dosiermengen-Einstellvorrichtung eingestellt werden kann. Nach Maßgabe der Dosiermengeneinstellung wird beim Niederdrücken eines Betätigungsknopfes der Inhalt der Spritze in einer voreingestellten Menge aus der Spritze ausgedrückt. Die Dosiermengen-Einstellvorrichtung besteht aus einem drehbaren Handknopf, der an dem Ende des stiftförmigen Gehäuses angebracht ist. Ferner ist an dem Gehäuse ein Display vorgesehen, an dem die eingestellte Dosiermenge mit einer Skala oder einer LCD-Anzeige angezeigt wird.

Es besteht die Schwierigkeit, dass sehbehinderte Personen nur mit äußerster Mühe in der Lage sind, die Dosiermenge einzustellen oder zu kontrollieren. Daher kann es zu Fehldosierungen kommen.

Eine Injektionsvorrichtung ist beschrieben in WO 99/02210. Diese Injektionsvorrichtung weist einen Pen mit stiftförmigem Gehäuse auf, der eine handbetätigbare Dosiermengen-Einstellvorrichtung aufweist. Zu diesem Gehäuse ist ein passendes Anzeigegerät in Form einer Kappe vorgesehen und an dem Anzeigegerät ist ein entsprechendes Display vorgesehen, an dem die am Pen vorgenommene Dosiermengeneinstellung abgelesen werden kann.

US 5,720,733 beschreibt ein Blutzucker-Messgerät, das anhand eines Blutstropfens den Blutzucker eines Patienten bestimmt, so dass der Patient die erforderliche Insulindosis, die zu injizieren ist, bestimmen kann. Die Insulinspritze enthält eine Elektrodenanordnung, die einen Kapazitätswert bildet, welcher der in der Spritze enthaltenen Dosis entspricht. Die Elektrodenanordnung wird mit entsprechenden Kontakten des Messgerätes in Berührung gebracht, so dass das Messgerät eine Information über die Höhe der in der Spritze verfügbaren Dosis erhält. Das Messgerät kann also lediglich angeben, ob die verfügbare Dosis der errechneten benötigten Dosis entspricht. Einstellungen kann der Benutzer dagegen nicht vornehmen und auch nicht durch eine vergrößerte Anzeige überprüfen.

Der Erfindung liegt die Aufgabe zugrunde, eine Injektionsvorrichtung mit stiftförmigem Gehäuse (Pen) zu schaffen, die in Kombination mit einem Blutzucker-Messgerät eine einfache Ablesung der eingestellten Dosiermenge auch für sehbehinderte Personen ermöglicht.

Die Lösung dieser Aufgabe erfolgt erfindungsgemäß mit den im Anspruch 1 angegebenen Merkmalen.

Bei der erfindungsgemäßen Injektionsvorrichtung wird das stiftförmige Gehäuse des Pens, das ein eigenes Display aufweist, mit einem externen Anzeigegerät gekoppelt, welches Informationen über die jeweils eingestellte Dosiermenge empfängt und die Dosiermenge an einer relativ großformatigen Anzeige anzeigt. Dadurch ist die Größe der Anzeigevorrichtung nicht auf das schmale Penformat des stiftförmigen Gehäuses beschränkt. Andererseits können die Vorteile des stiftförmigen Gehäuses, nämlich die einfache Mitführbarkeit und Handhabbarkeit, beibehalten werden. Lediglich zum Einstellen der Dosiermenge wird das stiftförmige Gehäuse an das Anzeigegerät angekoppelt.

Für das Anzeigegerät wird eine vorhandene Technik eingesetzt werden. Bei insulinspritzenden Diabetikern wird das Display des vorhandenen Blutzucker-Messgerätes für die Anzeige der Dosiermengeneinstellung benutzt. Durch die Verwendung des externen Displays kann auf die Verwendung von Batterien im Einmal-Pen verzichtet werden. Das Anzeigegerät kann auch ein Sprachmodul zur akustischen Angabe der eingestellten Dosierung enthalten. Die eingestellte Dosis wird mit Zeit, Datum und Art des Medikaments (z.B. Insulinzubereitung) abgespeichert.

Jedes Anzeigegerät kann Speichermöglichkeiten für mehrere Personen enthalten. Die betreffende Person wird anhand eines definierten Merkmals erkannt. Im Anzeigegerät sind den einzelnen stiftförmigen Gehäusen (Pens) jeweils individuelle Inhaltsstoffe (Insulinzubereitungen) zugeordnet. Das Anzeigegerät kann weitere personenbezogene Daten enthalten. Für Diabetiker können z.B. die Broteinheiten, der ermittelte Insulinbedarf zur Abdeckung einer Broteinheit, individuelle Therapieziele bei der Blutzuckerkontrolle, Stoffwechselbesonderheiten und die Wirkprofile der unterschiedlichen Insulinzubereitungen eingegeben werden oder von einem PC übernommen werden. Die externe Anzeige kann aufgrund ihrer Speichermöglichkeiten dem Arzt oder der Pflegekraft genaue Auskunft über die Art und den Zeitpunkt der Injektion geben. Aus den einer Person zugeordneten Daten kann anhand eines gemessenen Blutzucker-Wertes ein Vorschlag für die Insulindosierung berechnet werden. Diese Dosierung wird anschließend unter Verwendung desselben Anzeigegerätes am Pen eingestellt.

Der Erfindung liegt die Idee zugrunde, die Dosiseinstellung eines Pens durch Verwendung eines separaten Displays zu erleichtern. Der Datentransfer zwischen Pen und Anzeigegerät ermöglicht neben der optischen Anzeige der Dosiseinstellung weitere Funktionen, nämlich die akustische Anzeige der Dosiseinstellung, die Speicherung der Injektionsmenge mit Uhrzeit und Datum, beleuchtetes Display, Speicherung der injizierten Präparation und damit insgesamt ein automatisches Datenmanagement.

Die Datenübertragung von dem Pen zu dem Anzeigegerät kann auf unterschiedliche Weise erfolgen, beispielsweise elektrisch, optisch oder mechanisch. Es ist dabei möglich, den Pen direkt an das Gehäuse des Anzeigegeräts anzuklemmen oder das Anzeigegerät mit einem Stecker zu versehen, der an den Pen angesteckt wird. In jedem Fall sollte eine Unverwechselbarkeitsvorrichtung vorgesehen sein, die das Koppeln von Pen und Anzeigegerät nur mit der korrekten Positionierung (Polung) zuläßt.

Es ist auch möglich, den Pen mit einer von dem Anzeigegerät erkennbaren Kodierung auszustatten, so daß das Anzeigegerät stets weiß, mit welchem Pen bzw. Pentyp es zusammenarbeitet.

Es besteht die Möglichkeit, das Anzeigegerät und/oder den Pen mit einer Spannungsversorgung zu versehen, wobei die Spannungsversorgung des einen Gerätes das andere Gerät mitversorgen kann.

Im folgenden werden unter Bezugnahme auf die Zeichnungen Ausführungsbeispiele der Erfindung näher erläutert.

Es zeigen:
- Fig. 1: eine schematische perspektivische Darstellung der Injektionsvorrichtung mit Pen und Anzeigegerät bei elektrischer Kopplung,
- Fig. 2: eine Stirnansicht von Pen und Anzeigegerät im gekoppelten Zustand,
- Fig. 3: eine zweite Ausführungsform der Injektionsvorrichtung mit optischer Kopplung,
- Fig. 4: eine Stirnansicht von Fig. 3,
- Fig. 5: eine dritte Ausführungsform der Injektionsvorrichtung mit mechanischer Kopplung,
- Fig. 6: eine Seitenansicht der Kopplungsseite des Anzeigegerätes von Fig. 5,
- Fig. 7: eine Einzelheit bei der Ankopplung des Anzeigegerätes mit einem Adapterkabel,
- Fig. 8: eine vierte Ausführungsform der Injektionsvorrichtung, bei der das Anzeigegerät die Dosierungseinstellung am Pen bewirkt, und
- Fig. 9: eine Seitenansicht der Kopplungsseite des Anzeigegerätes von Fig. 8.

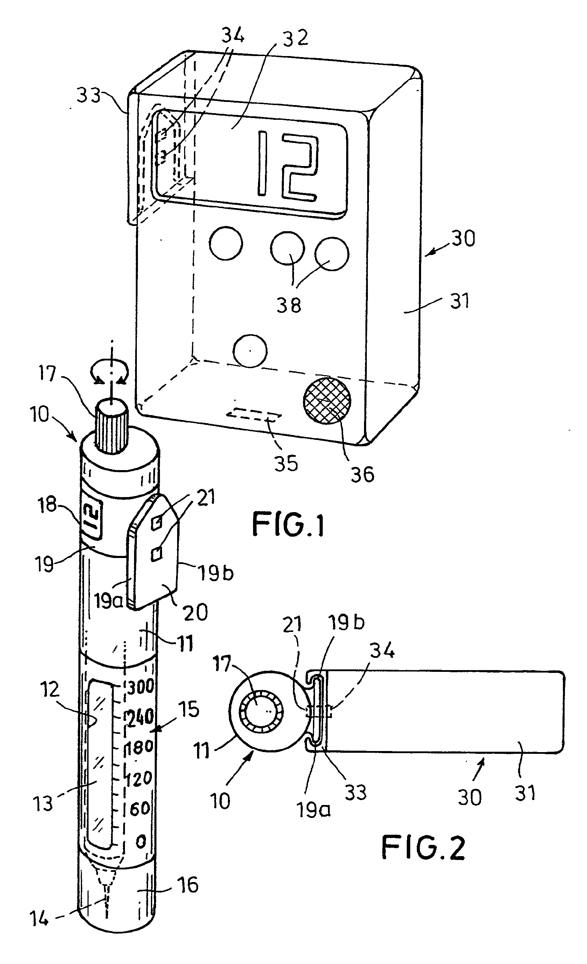

In den Fign. 1 und 2 ist ein Pen 10 dargestellt, welcher ein dünnes langgestrecktes stiftförmiges Gehäuse 11 aufweist. An der Wand des Gehäuses 11 ist ein Fenster 12 vorgesehen, durch das ein Teil einer in das Gehäuse 11 eingesetzten vorgefüllten Kartusche 13 sichtbar ist. Diese Kartusche enthält einen Zylinder und einen darin bewegbaren Kolben. Aus der Kartusche 13 wird ein Medikament, z.B. Insulin, ausgedrückt. Zu diesem Zweck weist die Kartusche 13 am vorderen Ende eine Injektionsnadel 14 auf. An dem Fenster 12 ist eine Skala 15 vorgesehen, an der die in der Kartusche enthaltene Restmenge abgelesen werden kann.

Am vorderen Ende ist das Gehäuse 11 mit einer abnehmbaren Schutzkappe 16 versehen. Wenn die Schutzkappe 16 abgenommen ist, liegt die Injektionsnadel 14 frei. Der Benutzer kann dann die Injektionsnadel 14 in den Körper einstechen und anschließend eine vorgewählte Menge an Flüssigkeit aus der Spritze 13 ausdrücken.

Die Dosiermenge wird an einer Dosiermengen-Einstellvorrichtung 17 eingestellt. Durch Drehen dieses Knopfes kann diejenige Flüssigkeitsmenge, die bei einem Ausdrückvorgang aus der Spritze 13 ausgedrückt wird, eingestellt werden. Durch Drehen des Knopfes wird das Maß des Vorschiebens eines Kolbens bei einem Auspreßvorgang verändert. Die Bewegung des Kolbens erfolgt durch Federkraft. Das Ausdrücken der Flüssigkeit aus der Spritze erfolgt durch Drücken eines seitlich an dem Gehäuse 11 vorgesehenen Betätigungsknopfes.

Das Volumen, das an der Dosiermengen-Einstellvorrichtung für einen Auspreßvorgang eingestellt worden ist, wird an einem Display 18 angezeigt, welches an dem Gehäuse 11 vorgesehen ist.

Dieses Display ist beispielsweise ein LCD-Display, an dem das Volumen in Millilitern angegeben wird. Das Gehäuse 11 enthält eine Batterie für die Stromversorgung des Displays 18.

Im rückwärtigen Bereich des Gehäuses 11 ist an dem Gehäuse eine längslaufende Linearführung 19 vorgesehen. Zwischen den Führungsschienen 19a,19b dieser Linearführung befindet sich eine Abflachung 20 und im Bereich dieser Abflachung 20 sind Kontakte 21 vorgesehen.

In Kombination mit dem Pen 10 ist das Anzeigegerät 30 vorgesehen. Es handelt sich um ein separates Gerät mit einem Gehäuse 31, das ein großformatiges Display 32 aufweist. An einer Schmalseite des Gehäuses 31 befindet sich eine flache Aufnahme 33, die mit einer Linearführung 19 des Pens 10 passend zusammengreifen kann. In der Aufnahme 33 sind Gegenkontakte 34 angeordnet, die mit den Kontakten 21 des Pens zusammenpassen. Somit kann das Gehäuse 31 seitlich auf den Pen 10 aufgeschoben werden, wobei die Kontakte 21 mit den Gegenkontakten 34 in Eingriff kommen. Die Linearführung 19 verläuft parallel zur Längsachse des Pens 10, so daß das Anzeigegerät 30 in Längsrichtung des Pens auf diesen aufgeschoben wird, wobei beide Teile dann eine starre Einheit bilden.

Die Dosiermengen-Einstellvorrichtung 17 enthält einen (nicht dargestellten) Kodierer, der ein elektrisches Signal, welches die eingestellte Dosiermenge repräsentiert, an die Kontakte 21 legt. Dieses Signal wird über die Gegenkontakte 34 zu dem Anzeigegerät 30 übertragen und - ggf. nach entsprechender Umwandlung - an dem großen Display 32 angezeigt. Somit kann der Benutzer an dem Display 32 die eingestellte Injektionsmenge leicht ablesen, ohne daß er auf das am Pen 10 vorgesehene kleine Display 18 angewiesen ist. Die Anzeige kann in ml oder auch in (Insulin)-Einheiten dargestellt werden.

Eine andere Möglichkeit der elektrischen Signalübertragung besteht darin, daß während der Dosiseinstellung am Pen bei jeder Dosiseinheit im Pen ein Impuls (oder eine Impulslücke) erzeugt wird. Die Anzahl der so erzeugten Impulse wird auf dem Display 32 dargestellt. Die Anzeigevorrichtung 30 hat eine eigene Spannungsversorgung, die an die Kontakte 21 des Pens gelegt wird, wobei die Betätigung der Einstellvorrichtung 17 entsprechende Impulse erzeugt. Die korrekte Nullstellung im Pen 10 zu Beginn der Dosiseinstellung erkennt das Anzeigegerät 30 mit Hilfe eines (nicht dargestellten) dritten Kontaktes, der nur in dieser Position leitend ist, oder an einem definierten Spannungsabfall (Übergangswiderstand), der charakteristisch für die Nullstellung ist. Korrekturen der Dosiseinstellung erfolgen durch Drehen der Einstellvorrichtung 17. Um die Drehrichtung zu erkennen, besteht das Signal für jede Einheit aus zwei unterschiedlichen Impulsen (kurz/lang oder schwach/stark). Die zeitliche Abfolge der beiden unterschiedlichen Impulse läßt die Drehrichtung erkennen.

Die Anzeigevorrichtung 30 ist im vorliegenden Fall zugleich ein Blutzucker-Testgerät, das generell so ausgebildet ist wie das Gerät Omnitest Sensor der Firma B. Braun Petzold GmbH. Dieses Blutzucker-Meßgerät weist einen Einführschlitz 35 zum Einschieben eines mit einer Blutprobe versehenen Teststreifens auf. An dem Display 32 wird dann der Blutzuckerwert angezeigt. In Kombination mit dem Pen 10 wird das Blutzucker-Meßgerät nunmehr auch als Anzeigegerät für die Dosisanzeige des Pens mitbenutzt. Somit ist ein zusätzliches Anzeigegerät entbehrlich.

An dem Gehäuse 31 des Anzeigegeräts 20 ist ferner ein Lautsprecher 36 vorgesehen. Das Gerät enthält einen Voice Processor, der die Dosisanzeige in eine gesprochene akustische Angabe umwandelt, so daß die Dosisanzeige auch hörbar gemacht wird.

An dem Gehäuse 31 kann ein Schieber vorgesehen sein, der über die Aufnahme 33 geschoben werden kann und diese im geschlossenen Zustand bedeckt, so daß die Gegenkontakte 34 geschützt untergebracht sind. Die Abdeckung öffnet automatisch beim Einschieben des Pens und schließt automatisch wieder beim Entfernen des Pens. Außerdem weist das Gehäuse 31 noch verschiedene Bedienknöpfe 38 auf.

Die mechanische Ankopplung des Pens 10 an ein externes Anzeigegerät 30 erfordert auf seiten des Pens eine asymmetrische Formgebung, um eine fehlerfreie Positionierung des Anzeigegerätes 30 zu gewährleisten. Das Anzeigegerät ist so ausgebildet, daß nach dem Einrasten des Pens 10 die Dosiermengen-Einstellvorrichtung 17 leicht zugänglich bleibt. Das Ankoppeln des Pens ist mit und ohne Schutzkappe 16 möglich. Das Display 18 des Pens sollte auch bei angekoppeltem Anzeigegerät 30 sichtbar bleiben.

Zur Erkennung des jeweiligen Typs des Pens können an dem Pen entsprechende Kodierungen angebracht sein, beispielsweise in Form von Erhöhungen oder Vertiefungen. Sie können auch durch herausbrechbare Laschen gebildet werden oder durch leitende Kontakte an der Oberfläche des Pens, die durch elektrische Kontakte des Anzeigegeräts detektiert werden.

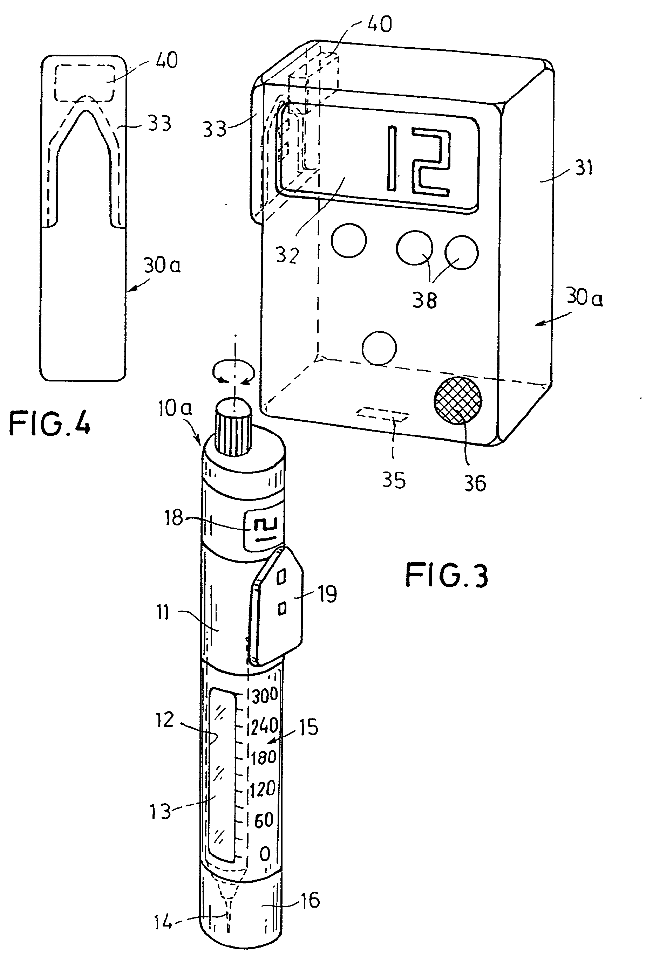

Das in den Fign. 3 und 4 dargestellte Ausführungsbeispiel verwendet einen geringfügig geänderten Pen 10a. Bei dem Pen 10a ist nämlich das Display 18 angrenzend an die Linearführung 19 angeordnet, daß sie von dem Anzeigegerät 30a bedeckt wird. Die Übermittlung des eingestellten Dosiswertes vom Pen 10a zum Anzeigegerät 30a erfolgt auf optischem Wege. An dem Anzeigegerät 30a ist ein optischer Sensor 40 vorgesehen, der dem Display 18 direkt gegenüberliegt. Daraufhin wird die gleiche Anzeige im Display 32 erzeugt.

Eine andere Möglichkeit besteht darin, daß der Sensor 40 zugleich einen Lichtsender enthält, der Licht auf das Display 18 fallen läßt. Während der Dosiseinstellung am Pen 10a wechseln bei jeder Einheit die Reflexionseigenschaften im Sichtfenster des Displays 18. Die wechselnde Reflexion wird vom Sensor 40 detektiert.

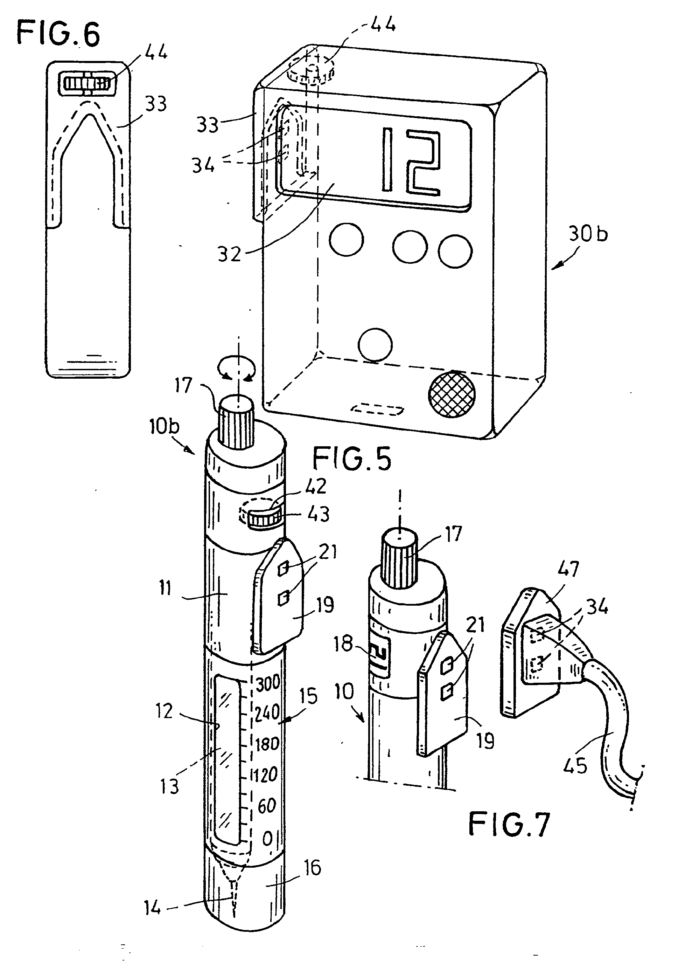

Bei dem Ausführungsbeispiel der Fign. 5 und 6 erfolgt die Übertragung des Dosiswertes mechanisch. Hierbei ist an dem Pen ein Schlitz 42 vorgesehen, der einen Teil des Umfangs eines Zahnrades 43 freilegt. Ein weiteres Zahnrad 44 ist in dem Anzeigegerät 30b so gelagert, daß ein Teil seines Umfangs in die Aufnahme 33 hineinragt. Beim Ankoppeln des Anzeigegeräts 30b an den Pen 10b gelangen die Zahnräder 43 und 44 in Eingriff. Die Drehbewegung der Einstellvorrichtung 17 wird auf diese Weise durch mechanisch zusammengreifende Kopplungsmittel in Form der Zahnräder 43,44 zum Anzeigegerät 30b übertragen.

Bei dem Ausführungsbeispiel von Fig. 7 ist der Pen 10 in gleicher Weise ausgebildet wie in Fig. 1, jedoch wird das Gehäuse des Anzeigegerätes 30 nicht unmittelbar an das Gehäuse des Pens angekuppelt. Vielmehr weist das Anzeigegerät 30 ein Kabel 45 auf, mit einem Stecker 47, der die Gegenkontakte 34 enthält, welche mit den Kontakten 21 des Pens verbunden werden. Es ist daher nicht nötig, das Gehäuse des Anzeigegerätes anzuheben und an den Pen anzukuppeln. Eine ähnliche Ankopplung eines separaten Gehäuses an einen Pen ist auch bei der optischen Abtastung gemäß Fig. 3 möglich. Dort kann der Sensor 40 in einem Stecker untergebracht sein, der über ein Lichtleitkabel oder auch elektrisch mit dem Gehäuse des Anzeigegeräts verbunden ist.

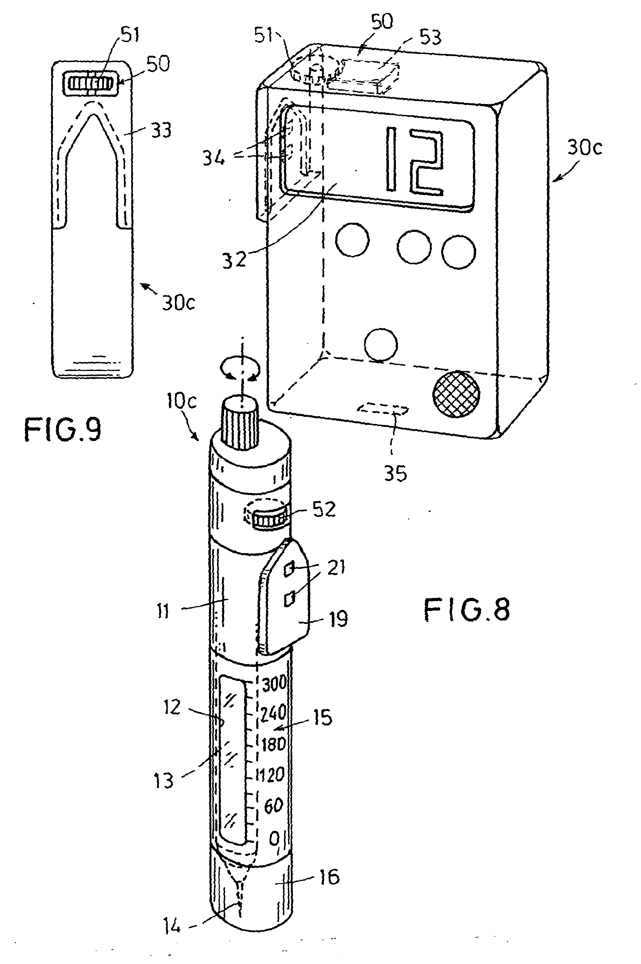

In den Fign. 8 und 9 ist ein Ausführungsbeispiel dargestellt, bei dem das Anzeigegerät 30c eine Dosiermengen-Einstellvorrichtung 50 enthält. Das Anzeigegerät 30c ist auch hier ein Blutzucker-Meßgerät mit einem Schlitz 35 zum Einschieben eines Meßstreifens. Aus dem vom Meßstreifen abgeleiteten Testergebnis und den patientenbezogenen Daten, die von Hand eingegeben werden, ermittelt das Anzeigegerät 30c die einzustellende Dosismenge an Insulin. Wenn das Anzeigegerät 30c an den Pen 10c angekuppelt wird, gelangt ein Zahnrad 51 der Dosiermengen-Einstellvorrichtung 50 in Eingriff mit einem Zahnrad 52 des Pens 10c. Durch Drehung des Zahnrades 52 wird im Pen 10c die Dosiermenge eingestellt, die von dem Anzeigegerät 30c ermittelt wurde. Das Zahnrad 51 des Anzeigegeräts 30c wird von einem Motor 53 angetrieben, der vorzugsweise ein Schrittschalt-Motor ist.

## Patentansprüche

1. Injektionsvorrichtung mit einem Pen (10) mit stiftförmigem Gehäuse (11), das eine Kartusche (13) mit einem in einem Zylinder der Kartusche bewegbaren Kolben enthält und eine handbetätigbare Dosiermengen-Einstellvorrichtung (17) zum Einstellen des Kolbens sowie ein Display (18) aufweist, und mit einem externen Anzeigegerät (30,30a,30b,30c), das an das stiftförmige Gehäuse (10) derart anschließbar ist, dass es Informationen über die jeweils eingestellte Dosiermenge empfängt, wobei das Anzeigegerät (30,30a,30b,30c) ein Blutzucker-Messgerät mit einem Display (32) mit gegenüber dem Display (18) des Pens vergrößerter Anzeige ist, dessen Display für eine zusätzliche Anzeige der Dosiermengeneinstellung benutzbar ist.

2. Injektionsvorrichtung nach Anspruch 1, **dadurch gekennzeichnet, dass** das Anzeigegerät (30,30a,30b,30c) ein Gehäuse (31) aufweist, welches mit einer Aussparung (33) zum Einsetzen des stiftförmigen Gehäuses (11) des Pens (10a,10b,10c) versehen ist.

3. Injektionsvorrichtung nach Anspruch 1 oder 2, **dadurch gekennzeichnet, dass** die Informationsübertragung zwischen dem stiftförmigen Gehäuse (11) und dem Anzeigegerät (30) elektrisch erfolgt.

4. Injektionsvorrichtung nach Anspruch 1 oder 2, **dadurch gekennzeichnet, dass** das Anzeigegerät (30a) einen auf das Display (18) des Pens (10) reagierenden optischen Sensor (40) enthält.

5. Injektionsvorrichtung nach Anspruch 1 oder 2, **dadurch gekennzeichnet, dass** die Informationsübertragung zwischen dem stiftförmigen Gehäuse (11) und dem Anzeigegerät (30b) durch mechanisch zusammengreifende Kopplungsmittel (43,44) erfolgt.

6. Injektionsvorrichtung nach einem der Ansprüche 1-5, **dadurch gekennzeichnet, dass** der Pen oder das Anzeigegerät keine eigene Stromversorgung enthält und von der jeweils anderen Vorrichtung mitversorgt wird.

7. Injektionsvorrichtung nach einem der Ansprüche 1-6, **dadurch gekennzeichnet, dass** das Anzeigegerät (30,30a,30b,30c) eine akustische Ansage der eingestellten Dosiermenge aufweist.

8. Injektionsvorrichtung nach einem der Ansprüche 1-7, **dadurch gekennzeichnet, dass** das Anzeigegerät einen Detektor aufweist, der definierte Merkmale eines Pens erkennt und **dadurch** den Pen identifiziert, womit Informationen über die verwendete Zubereitung des Medikaments und die Zuordnung zu einem bestimmten Patienten verbunden sind.

9. Injektionsvorrichtung nach einem der Ansprüche 1-8, **dadurch gekennzeichnet, dass** zu Beginn der Dosiseinstellung die korrekte NullStellung abgeprüft wird.

10. Injektionsvorrichtung nach einem der Ansprüche 1-9, **dadurch gekennzeichnet, dass** neben der Dosismenge auch weitere Parameter, wie Zeit, Datum, Patient, individuelle Patientendaten, abgespeichert werden.

11. Injektionsvorrichtung nach einem der Ansprüche 1-10, **dadurch gekennzeichnet, dass** das Anzeigegerät zur Datenübertragung mit einem externen Rechner verbindbar ist.

## Claims

1. An injection device comprising a pen (10) with a pin-shaped housing (11) including a cartridge (13) with a piston movable within a cylinder of said cartridge and including a manually operable dose setting device (17) to adjust the piston and a display (18), and an external display apparatus (30, 30a, 30b, 30c) adapted to be connected to the pin-shaped housing (11) such that it receives information about the respective dose set, wherein the display apparatus (30, 30a, 30b, 30c) is a blood sugar measuring device with a display (32) whose indication is larger that that of the pen's display (18), the display thereof being adapted for use as an additional indication of the dose setting.

2. The injection device of claim 1, wherein the display apparatus (30, 30a, 30b, 30c) comprises a housing (31) provided with a cutout (33) for inserting the pin-shaped housing (11) of the pen (10a, 10b, 10c).

3. The injection device of claim 1 or 2, wherein information is transferred electrically between the pin-shaped housing (11) and the display apparatus (30).

4. The injection device of claim 1 or 2, wherein display apparatus (30a) comprises an optical sensor (40) responding to the display device (18) of the pen (10).

5. The injection device of claim 1 or 2, wherein information is transferred between the pin-shaped housing (11) and the display apparatus (30b) by mechanically engaging coupling means (43, 44).

6. The injection device of one of claims 1-5, wherein the pen or the display apparatus includes no power supply of its own and is supplied by the respective other device.

7. The injection device of one of claims 1-6, wherein the display apparatus (30, 30a, 30b, 30c) comprises an acoustic indication of the dose set.

8. The injection device of one of claims 1-7, wherein the display apparatus comprises a detector recognizing defined features of a pen and identifying the pen thereby, information about the preparation of a medication used and the relation to a certain patient being associated with these features.

9. The injection device of one of claims 1-8, wherein the correct zero position is checked at the beginning of the dose setting operation.

10. The injection device of one of claims 1-9, wherein, besides the dose, other parameters such as time, date, patient, and individual patient data are stored.

11. The injection device of one of claims 1-10, wherein the display apparatus may be connected to an external computer for data transfer.

## Revendications

1. Injecteur comprenant un stylo (10) avec un boîtier en forme de crayon (11) qui contient une cartouche (13) avec un piston mobile dans un cylindre de la cartouche et un dispositif de dosage manuel (17) pour régler le piston ainsi qu'un écran (18), et avec un afficheur externe (30, 30a, 30b, 30c) qui peut être raccordé au boîtier en forme de crayon (10) de sorte qu'il reçoit des information sur le dosage réglé, l'afficheur (30, 30a, 30b, 30c) étant un glucomètre avec un écran (32) offrant un affichage plus grand que l'écran (18) du stylo, dont l'écran peut être utilisé pour offrir un affichage supplémentaire du dosage.

2. Injecteur selon la revendication 1, **caractérisé en ce que** l'afficheur (30, 30a, 30b, 30c) possède un boîtier (31) qui est doté d'un évidement (33) pour insérer le boîtier en forme de crayon (11) du stylo (10a, 10b, 10c).

3. Injecteur selon la revendication 1 ou 2, **caractérisé en ce que** le transfert d'informations entre le boîtier en forme de crayon (11) et l'afficheur (30a) se fait électriquement.

4. Injecteur selon la revendication 1 ou 2, **caractérisé en ce que** l'afficheur (30a) contient un capteur optique (40) réagissant à l'écran (18) du stylo (10).

5. Injecteur selon la revendication 1 ou 2, **caractérisé en ce que** le transfert d'informations entre le boîtier en forme de crayon (11) et l'afficheur (30a) se fait par des moyens de couplage mécaniques (43, 44).

6. Injecteur selon une des revendications 1 à 5, **caractérisé en ce que** le stylo ou l'afficheur ne contient pas d'alimentation électrique propre et est alimenté par celle de l'autre dispositif.

7. Injecteur selon une des revendications 1 à 6, **caractérisé en ce que** l'afficheur (30, 30a, 30b, 30c) offre une annonce acoustique du dosage réglé.

8. Injecteur selon une des revendications 1 à 7, **caractérisé en ce que** l'afficheur possède un détecteur qui identifie les caractéristiques définies d'un stylo et identifie ainsi le stylo auquel sont liées des informations sur la préparation du médicament et l'attribution à un patient déterminé.

9. Injecteur selon une des revendications 1 à 8, **caractérisé en ce que** le bon réglage du zéro est vérifié au début du dosage.

10. Injecteur selon une des revendications 1 à 9, **caractérisé en ce qu'**outre la dose, d'autres paramètres tels que l'heure, la date, l'identité du patient et des données individuelles sur le patient sont également mémorisés.

11. Injecteur selon une des revendications 1 à 10, **caractérisé en ce que** l'afficheur peut être relié à un ordinateur pour transmettre des données.
